# EUROPEAN PATENT SPECIFICATION

(11) **EP 0 613 559 B1**
(45) Date of publication and mention of the grant of the patent: **30.07.1997**
(21) Application number: 92923335.1
(22) Date of filing: 10.11.1992
(51) Int. Cl.: G01N 33/558, G01N 33/543

(54) **LIQUID TRANSFER ASSAY DEVICES**
TRANSFER-TESTVORRICHTUNG FUR FLUSSIGKEITEN.
DISPOSITIFS DE DOSAGE A TRANSFERT DE LIQUIDE

(30) Priority: 11.11.1991 GB 9123903
(43) Date of publication of application: 07.09.1994
(73) Proprietor: BRITISH TECHNOLOGY GROUP LIMITED, London SE1 6BU (GB)
(72) Inventor: Bunce, Roger, Abraham, Birmingham B30 1DZ (GB); STARSMORE, Stephen, John, Birmingham B29 4LT (GB)
(74) Representative: Parker, Geoffrey
(86) International application number: GB9202075
(87) International publication number: WO9310457

(56) References cited:
- WO-A-89/03992
- WO-A-90/11519
- WO-A-91/02589

## Description

There is considerable commercial interest in simple, disposable, self-contained devices for performing biochemical diagnostic assays in extra-laboratory situations. Ideally such devices should avoid any need for complex manual procedures, such as a timed sequence of reagent additions to an analyte, and so be suitable for use by lay persons.

According to Patent Specification WO90/11519 such devices can be of a kind comprising first and second liquid flow channels of porous materials leading from a respective pair of channel ends to a common site, which channels are operable to transfer liquid by capillary flow to the common site in sequentially timed manner following simultaneous application of the liquid to the pair of channel ends.

In one more particular form of this kind of device the first and second channels merge into, and transfer liquid in sequentially timed manner to a common channel. It follows that thc later one of the first and second channels to transfer liquid to the common channel will itself also receive liquid from the other channel. The one channel accordingly receives two opposite flows which merge. After such merging it is normally appropriate for liquid flow to continue through the one channel in the same direction as the initial flow therein. However, continuing flow from the other channel can act undesirably against this result.

An object of the present invention is to improve this last situation and to this end the invention provides a device for performing biochemical diagnostic assays comprising first and second liquid flow channels of porous material providing flow communication from a respective pair of channel ends to a common site, which channels are operable to transfer liquid by capillary flow to the common site in sequentially timed manner following simultaneous application of the liquid to the pair of channel ends, wherein said first and second channels interconnect and then both continue in ongoing separate flowpaths. Such an arrangement of channels can be equated with an electrical bridge circuit and, as such, conformed to produce a substantially predetermined flow condition through the channel interconection. At least part of the first and second channels may have hydraulic resistance in the longitudinal direction selected to provide said flow condition. One such condition of particular practical interest is that of null flow.

Preferably, said first channel and said second channel each comprise respectively separate portions upstream and downstream of the interconnection, and in each of the channels the ratio of the hydraulic resistance in the longitudinal direction in the upstream portion to that in the downstream portion may be substantially equal.

At least one of said first and second channels has a plurality of sub-channels arranged in parallel, and at least one transverse flow obstacle may be provided adjacent to the ends of said sub-channels. One of said transverse flow obstacles may be provided for every two said sub-channels.

In a preferred form of the invention, a third liquid flow channel is provided interconnecting with at least one of said first and second channels, said third channel being located such that liquid can be applied simultaneously to the channel ends of said first, second and third channels.

The invention is further described below, by way of example, with reference to the accompanying drawings, in which:-

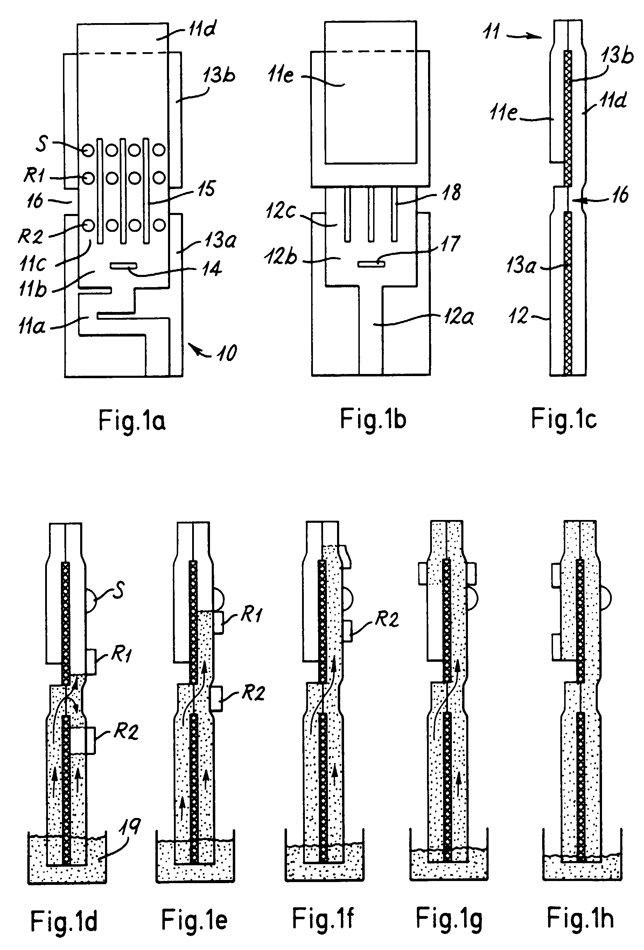

Figures 1 to 5 respectively illustrate different forms of device which accord with the invention and/or are explanatory of the operation of such a device.

The device of Figure 1 is of generally rectangular sheet overall form, denoted at 10, and is respectively illustrated in front, rear and side views at (a),(b) and (c).

The device has four pairs of first channels 11 and second channels 12 respectively formed by front and rear layers of porous material, which layers are separated by a liquid impervious membrane 13.

The first channels 11 commence from one end of the device in a common portion 11a of narrowed tortuous form. Thereafter the channels assume a broadened rectilinear form having successive portions 11b,c and d. The portion 11b has partway therealong a liquid impermeable bar 14 extending transversely across its central region and portion 11c has three uniformly spaced, parallel, liquid impermeable bars 15 extending therealong to define four discrete first channel portions. Each of these last portions has located successively therealong two reagent regions R2 and R1 and a sample application region S.

The membrane 13 is in two portions 13a and b. The first portion 13a extends against first channel portions 11a,b and c up to a point beyond reagent zones R2, but not as far as reagent zones R1. The second membrane portion 13b extends against first channel portions 11c and d from a point just before reagent zones R1 in the former, but without engaging the first membrane portion. There is accordingly a transverse gap 16 between the membrane portions.

The second channels 12 commence from the same end of the device as the first channels in a common portion 12a of narrowed rectilinear form. Thereafter the channels assume a broadened rectilinear form having successive portions 12b and c. These last portions are similar to the first channel portions 11b and c and have respective bars 17 and 18. However, the discrete second channel portions 12c terminate at the gap where they respectively engage the first channel portions 11c.

It will be noted that, as shown, the first channel portion 11d can effectively continue into a portion 11e formed as a part-layer on the other face of the device, with the portions 11d and e being mutually engaged beyond the end of membrane portion 13b.

In practical construction the porous material is suitably of filter paper such as Millipore (Trademark) AP25, and the bars within this material are formed by excision to form slots or by impregnation of wax or other liquid impermeable material. The membrane portions are of any suitable sheet plastics material bonded by adhesive with the porous material. The porous material layers are also bonded together, where they engage at gap 16, and between portions 11a and e, but in such a way as to allow liquid communication by capillary action.

The reagent regions R1 and R2 each contain lyophilised reagent materials impregnated into the porous material. For a typical immunoassay, R1 would be an enzyme-labelled antibody, and R2 a colorimetric substrate. The sample region S would incorporate antibodies, to the antigen of interest in the sample, immobilised onto the porous material. Each discrete channel may contain similar or different reagents and be related to similar or different samples.

In a typical use of the device, a biolgical sample, such as serum or urine, is applied to the sample regions S and antigen in the sample begins to bind to the immobilised antibody. The lower end of the device is then immersed into liquid 19 and this flows up the first and second channels as shown by Figure 1(d). Flow through the first channel portion 11a will take longer than that through the second channel portion 12a because of the tortuous form of the former. In the channel portions 11b and 12b liquid flow is similarly deflected around the respective bars 14 and 17 so as to arrive at the associated discrete channel portions 11c and 12c uniformly in each layer. Liquid flow in the second channels will reach the gap 16 first and passes into the first channels to continue upwardly and downwardly in the latter.

When this upward flow reaches region R1 the associated reagents are reconstituted and carried along with the upward flow. Also the downward flow proceeds to meet the initial upward flow in the first channels, these flows saturate the channels when they meet and then continue as an upward flow carrying along the reconstituted reagents from regions R2. These reagent movements are indicated by Figure 1(e).

Reagent R1 reaches the sample region S and a portion binds to the immobilised sample. The time between this binding and the earlier application of the sample to the region S allows the sample to incubate. Unbound material is washed away from region S by the continuing upward liquid flow. This phase of operation is indicated by Figure 1(f).

Further flow then causes reagent R2 to flow through and react with that portion of reagent R1 bound at the sample region as indicated by Figure 1(g). An insoluble colour is produced, the intensity of which depends upon the amount of the antigen of interest in the sample.

Flow then continues through the sample region, which stabilises the colour, and into the first channel portions 11d and e which serve as a waste reservoir. Flow stops when this reservoir is saturated and the operation is complete. This final phase is indicated by Figure 11(h).

While there is reference above to first channel portions 11c,d and e, it will be appreciated that beyond the gap 16, where the first and second channels are in liquid flow communication, the portions are common to both channels.

In any event, the device of Figure 1 is of a beneficially compacted form by virtue of this inter-channel flow communication being effected between superposed layers of porous material in which the discrete first and second channel portions of a multiple device can be respectively formed.

However, the specific form of Figure 1 can be improved. A significant facet of the operational procedure is the separation of application of the two reagents at the sample region. The extent of this separation is determined, at least in part, by the width of the second channel portion 12a because this determines the volume flow rate of liquid through the second channels. If this flow rate is too large relative to that in the first channels, the former flow will be dominant at the gap 16 and the reagents R2 will move too slowly. If, in compensation, portion 12a is unduly narrowed, the time taken to saturate region 16 can be unacceptably long.

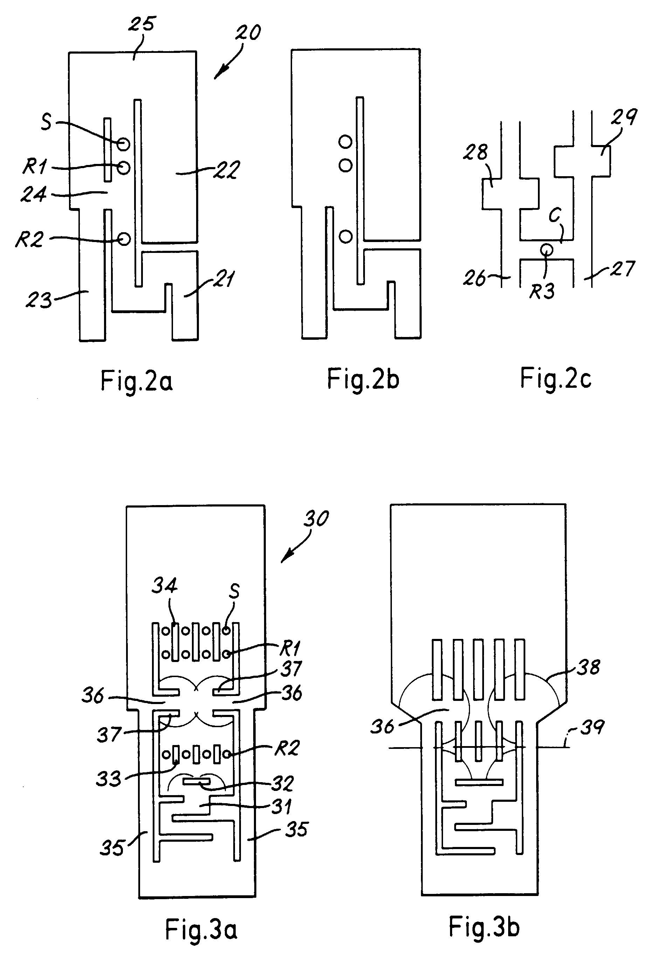

This difficulty can be avoided by use of a channel arrangement analogous to a balanced electrical bridge circuit as explained with reference to Figure 2.

Figure 2(a) shows a planar device denoted generally at 20 and involving a single pair of first and second channels formed by a single layer of porous material. In comparison with Figure 1 it will be seen that the planar device has a first channel 21 which starts from one end in a tortuous manner, and then proceeds rectilinearly along the device to its other end where it turns transversely and downwardly into a widened reservoir area 22. In its rectilinear part this channel has reagent and sample regions R1,R2 and S. A second channel 23 of rectilinear form starts from the same end as the first channel and passes in separated manner alongside the latter, except for a transverse interconnection 24 with the first channel between regions R1 and R2, and another 25 at the other end of the device. At the interconnection 24 the on-going second channel changes in width, by widening as shown, in order to modify the on-going resistance to flow relative to that in the preceding channel portion. The relevance of this will be appreciated further below.

Operation of this device will be largely evident from that given above for Figure 1. Sample is applied to region S and the lower ends of the channels immersed in liquid. Upward liquid flow occurs, but with a delay in the tortuous first channel portion, whereby flow occurs from the second channel, through connection 24, into the first channel. As before, reagents R1 and R2 are reconstituted and carried to the sample S in sequence, with waste products continuing to the reservoir 22.

A difference in this case is that the second channel flow additionally continues separately beyond connection 24 to pass through connection 25 to the waste reservoir.

Now hydraulic flow and pressure are analogous to electrical current and voltage, with hydraulic resistance to flow equating with electrical resistance. The hydraulic situation in Figure 2(a) can then be equated with a Wheatstone bridge circuit with the two channels representing the two sides of the bridge, and the connection 24 representing the cross connection in the bridge circuit. More specifically, the hydraulic resistances in the two channels up to and then beyond connection 24, represent the electrical resistances of the four arms of the bridge circuit and, when these resistances have the same proportions about the cross connection, there is zero flow across this connection. This is desirable for operation of the device of Figure 2(a) in that flow from the second channel cannot dominate that in the first channel following saturation below the connection 25.

Figure 2(b) illustrates a simplification in which the first and second channel portions above the connection 24 are not physically separated.

Figure 2(c) shows a modification of Figure 2(a) which causes flow direction to change or oscillate across the interconnection between the first and second flow channels. This is useful for automating diagnostic systems in which, for example, a sample 'visits' two analytical sites each sensitive to a specific analyte. Alternatively, a chemical may be made to 'oscillate' between two temperature zones, as in a polymerase chain reaction.

Referring to Figure 2(c), liquid flows up channels 26 and 27 and approaches chemical R3 from both ends of a transverse interconnection channel C. Meanwhile, the liquid continues into portions downstream of the interconnection channel where reservoirs 28 and 29 are provided in channels 26 and 27 respectively. When liquid flows into the first reservoir, for example 28 in Figure 2(c), the null flow in channel C is disturbed due to the resulting reduction in flow resistance in channel 26 and R3 is carried to the left along the channel. Subsequently, liquid flows into reservoir 29 in channel 27 and the balance of flow is again changed, moving R3 to the right along channel C. Further reservoirs may be added in series (not shown) to increase the number of cycles in this flow oscillation.

Figure 3 illustrates devices similar to those of Figure 2 in being of planar form from a single layer of porous material, but in this case they are also of multiple form involving plural pairs of channels.

The device of Figure 3(a) is denoted generally at 30. It has first channels 31 of similar form to those of Figure 1 in having a tortuous portion connected to a rectilinear portion which first has a transverse deflector bar 32 and then longitudinal channel separator bars 33. Thereafter the first channels rejoin in a common portion which is transversely necked in, as discussed below, and broadened again into a further portion with longitudinal channel separator bars 34.

The second channels 35 are deployed on both sides of the first channel array in an effective duplication of the unilateral arrangement in Figure 2(a), with cross connections 36 to the first channels intermediate reagent regions R1 and R2. These connections are defined by transverse channel separating bars 37 projecting from those which separate the first and second channels longitudinally. The bars 37 are of lengths such as to cause flow from the second channels to progress into the first channels, upwardly and downwardly, in a uniform manner.

Operation of this device will be evident from that described above for Figures 1 and 2. However, it is to be noted that when the cross connections 36 and the preceding channel portions are saturated, there is to be zero flow in the connections. In these circumstances reagents R2 are carried upwardly through the necked first channel portions, along curved streambands, and there is a tendency for the reagents to become diffuse as these bands become longer.

Figure 3(b) shows this last device modified in similar manner as Figure 2(b) relative to 2(a) by omission of the upper first and second channel separating bars and, in addition, the cross connection bars 37. At the same time the gap at connections 36 between the upper and lower channel portions is narrowed and the flow deflector bar 32 is widened. In the result the opposite flows towards reagents R2 have similar circular wavefronts indicated at 38 which will meet to form, at saturation, a temporary stagnation line 39 of generally rectilinear form across the device. The reconstituted reagents R2 will then move from this line uniformly upwards with no significant risk of diffusion.

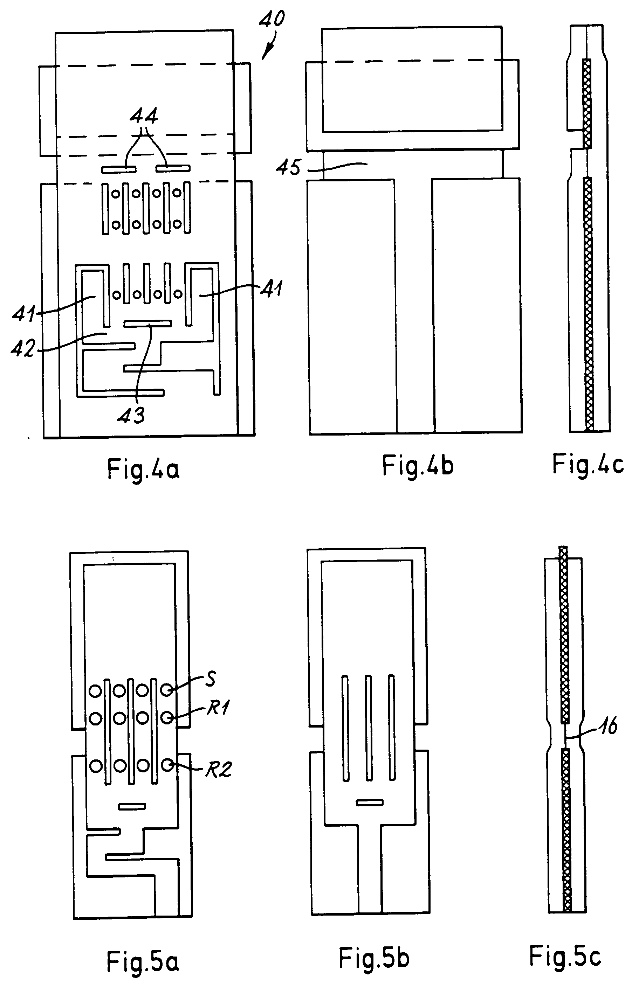

The bridge-like forms described above need to be fully wetted in order to balance effectively. The device 40 of Figure 4 is modified relative to that of Figure 3 to facilitate this result.

Figure 4 in fact involves two layers of porous material, somewhat like Figure 1, and so is shown in front, rear and side views respectively at (a),(b) and (c).

The front layer has a form corresponding to Figure 3(b) except for two additions.

The first addition involves the provision of two reservoirs 41 connected at 42 with the opposite sides of the first channel common portion which incorporates the transverse deflector bar 43. These reservoirs delay movement of reagent R2 during saturation of the bridge from below, particularly in relation to the relatively modest flow contributions from the second channels, and ensure uniformity of flow across the thickness of the porous material, which otherwise may tend to saturate at different rates on opposite surfaces. The extent of the time delay depends on the area of the reservoirs 41 and the size of the connections 42.

The second addition involves the provision of two further deflector bars 44 above the uppermost ends of the discrete first channels. These bars serve to apply flow uniformly to the channels in a downward direction and avoid a need for these channels to be unduly narrow.

This last flow is provided by the rear layer 45 which is of T-shape to convey liquid up to and across the front layer behind the deflector bars 44. This flow serves to ensure adequate saturation of the bridge from above.

The rear layer can also provide a separate portion to connect at the top of the device with the waste reservoir and so further compact the device as in Figure 1.

Turning lastly to the device of Figure 5, this is a modification of that of Figure 1 but which employs the benefits of a bridge.

As with Figure 1, the device of Figure 5 is shown in front, rear and side views respectively at (a),(b) and (c). Figure 5 in fact shows one difference, namely, that the waste reservoir does not continue from the front layer to the rear, but instead the second channels at the rear continue upwardly, after transverse engagement 16 with the first channels, into a common portion. The result, as seen in side view, is to form an effective bridge whereby, at saturation above, there is no flow between the first and second channels.

## Claims

1. A device (10; 20; 30; 40) for performing biochemical diagnostic assays comprising first and second liquid flow channels (11,12: 21,23; 31,35) of porous material providing flow communication from a respective pair of channel ends to a common site (S), which channels are operable to transfer liquid by capillary flow to the common site in sequentially timed manner following simultaneous application of the liquid to the pair of channel ends, wherein said first and second channels interconnect and then both continue in ongoing separate flowpaths.

2. A device according to claim 1, wherein said common site is located in one of the ongoing flowpaths.

3. A device according to claim 1 or 2, wherein said first and second channels are conformed to produce a substantially predetermined flow condition across the interconnection.

4. A device according to claim 3, wherein at least part of the first and second channels has hydraulic resistance in the longitudinal direction selected to provide said flow condition.

5. A device according to claim 4, wherein at least part of the first and second channels has cross sectional dimensions selected to provide the required hydraulic resistance.

6. A device according to any of claims 3 to 5, wherein said flow condition is null flow across the interconnection between said first and second channels when both channels are saturated at least as far as the interconnection.

7. A device according to any preceding claim, wherein said first channel and said second channel each comprise respectively separate portions upstream and downstream of the interconnection.

8. A device according to claim 7, wherein in each of the first and second channels the ratio of the hydraulic resistance in the longitudinal direction in the upstream portion to that in the downstream portion is substantially equal.

9. A device according to claim 7, wherein at least one of the downstream portions of said first and second channels comprise zones of different hydraulic resistance in the flow direction.

10. A device according to claim 9, wherein the zones of different hydraulic resistance are arranged to produce oscillating flow across the interconnection between said first and second channels.

11. A device according to any preceding claim, wherein at least one of said first and second channels has a plurality of sub-channels arranged in parallel.

12. A device according to claim 11, wherein at least one transverse flow obstacle is provided adjacent to the ends of said sub-channels.

13. A device according to claim 12, wherein one of said transverse flow obstacles is provided for every two said sub-channels.

14. A device according to any preceding claim, wherein said first and second channels comprise superposed layers of porous material separated by an impervious layer being omitted where said channels interconnect.

15. A device according to any of claims 1 to 13, wherein said device comprises a single sheet of porous material and said first and second channels are formed by providing impervious flow separating zones within said sheet.

16. A device according to claim 15, wherein said first channel extends from said channel end towards said common site in a portion disposed generally centrally of said sheet, and the second channel from said one end towards said common site extending in two side portion opposed about said central portion, said central portion and said side portions providing liquid flow from said one end to said common site in sequentially timed manner, wherein said side portions interconnect with said first channel in two zones transverse to said side portions and said side portions have downstream portions beyond said interconnection.

17. A device according to claim 16, wherein said device is conformed such that flows of liquid from the first and second channels meet in a substantially straight intersection front.

18. A device according to any preceding claim, wherein a third liquid flow channel is provided interconnecting with at least one of said first and second channels, said third channel being located such that liquid can be applied simultaneously to the channel ends of said first, second and third channels.

19. A device according to any preceding claim, wherein a flow delay reservoir is provided in at least one of said first and second channels.

## Patentansprüche

1. Vorrichtung (10; 20; 30; 40) zum Durchführen biochemischer diagnostischer Auswertungen mit ersten und zweiten Flüssigkeitsstromkanälen (11, 12; 21, 23; 31, 35) aus porösem Material, die von einem jeweiligen Paar Kanalenden zu einem gemeinsamen Ort (S) eine Stromverbindung schaffen, welche Kanäle betreibbar sind, um eine Flüssigkeit durch Kapillarstrom zu dem gemeinsamen Ort in aufeinanderfolgend zeitlich abgestimmter Weise nach gleichzeitiger Zuführung der Flüssigkeit zu dem Paar Kanalenden zu übertragen, worin die ersten und zweiten Kanäle miteinander verbunden sind und sich dann in weitergehenden separaten Stromwegen fortsetzen.

2. Vorrichtung nach Anspruch 1, worin der gemeinsame Ort in einem der weitergehenden Stromwege liegt.

3. Vorrichtung nach Anspruch 1 oder 2, worin die ersten und zweiten Kanäle angepaßt sind, um einen im wesentlichen vorbestimmten Stromzustand über die Verbindung zu erzeugen.

4. Vorrichtung nach Anspruch 3, worin zumindest ein Teil der ersten und zweiten Kanäle einen hydraulischen Widerstand in Längsrichtung aufweist, der so ausgewählt ist, daß er den Stromzustand liefert.

5. Vorrichtung nach Anspruch 4, worin zumindest ein Teil der ersten und zweiten Kanäle Querschnittsabmessungen hat, die so ausgewählt sind, daß sie den erforderlichen hydraulischen Widerstand liefern.

6. Vorrichtung nach einem der Ansprüche 3 bis 5, worin der Stromzustand ein Nullstrom über die Verbindung zwischen den ersten und zweiten Kanälen ist, wenn beide Kanäle zumindest bis zur Verbindung gesättigt sind.

7. Vorrichtung nach einem vorhergehenden Anspruch, worin der erste Kanal und der zweite Kanal jeweils stromaufwärts und stromabwärts der Verbindung jeweils separate Teile aufweisen.

8. Vorrichtung nach Anspruch 7, worin in jedem der ersten und zweiten Kanäle das Verhältnis des hydraulischen Widerstands in der Längsrichtung im stromaufwärtigen Teil zu dem im stromabwärtigen Teil im wesentlichen gleich ist.

9. Vorrichtung nach Anspruch 7, worin zumindest einer der stromabwärtigen Teile der ersten und zweiten Kanäle Zonen verschiedenen hydraulischen Widerstands in der Stromrichtung aufweist.

10. Vorrichtung nach Anspruch 9, worin die Zonen verschiedenen hydraulischen Widerstands angeordnet sind, um einen oszillierenden Strom über die Verbindung zwischen den ersten und zweiten Kanälen zu erzeugen.

11. Vorrichtung nach einem vorhergehenden Anspruch, worin zumindest einer der ersten und zweiten Kanäle eine Vielzahl parallel angeordneter Nebenkanäle aufweist.

12. Vorrichtung nach Anspruch 11, worin zumindest ein Querstromhindernis den Enden der Nebenkanäle benachbart vorgesehen ist.

13. Vorrichtung nach Anspruch 12, worin eines der Querstromhindernisse für alle zwei Nebenkanäle vorgesehen ist.

14. Vorrichtung nach einem vorhergehenden Anspruch, worin die ersten und zweiten Kanäle übereinandergelegte Schichten aus porösem Material aufweisen, die durch eine undurchlässige Schicht getrennt sind, die weggelassen ist, wo die Kanäle miteinander verbunden sind.

15. Vorrichtung nach einem der Ansprüche 1 bis 13, worin die Vorrichtung eine einzige Scheibe aus porösem Material aufweist und die ersten und zweiten Kanäle gebildet sind, indem undurchlässige stromtrennende Zonen innerhalb der Scheibe vorgesehen sind.

16. Vorrichtung nach Anspruch 15, worin sich der erste Kanal von dem Kanalende zum gemeinsamen Ort hin in einem Teil erstreckt, der im allgemeinen in der Mitte der Scheibe angeordnet ist, und wobei sich der zweite Kanal von dem einen Ende zum gemeinsamen Ort hin in zwei, beim zentralen Teil gegenüberliegenden Seitenteilen erstreckt, wobei der zentrale Teil und die Seitenteile einen Flüssigkeitsstrom von dem einen Ende zum gemeinsamen Ort in aufeinanderfolgend zeitlich abgestimmter Weise liefern, worin die Seitenteile mit dem ersten Kanal in zwei Zonen quer zu den Seitenteilen verbunden sind und die Seitenteile stromabwärtige Teile über die Verbindung hinaus aufweisen.

17. Vorrichtung nach Anspruch 16, worin die Vorrichtung derart angepaßt ist, daß sich Flüssigkeitsströme von den ersten und zweiten Kanälen in einer im wesentlichen geraden Kreuzungsfront treffen.

18. Vorrichtung nach einem vorhergehenden Anspruch, worin ein dritter Flüssigkeitsstromkanal vorgesehen ist, der mit zumindest einem der ersten und zweiten Kanäle verbunden ist, wobei der dritte Kanal derart gelegen ist, daß den Kanalenden der ersten, zweiten und dritten Kanäle gleichzeitig Flüssigkeit zugeführt werden kann.

19. Vorrichtung nach einem vorhergehenden Anspruch, worin ein Stromverzögerungsreservoir in zumindest einem der ersten und zweiten Kanäle vorgesehen ist.

## Revendications

1. Dispositif (10 ,20 ;30 ;40) pour effectuer des dosages biochimiques diagnostiques, comprenant un premier et un deuxième conduits (11,12;21,23,31,35) d'écoulement de liquide, en matière poreuse, assurant une communication de flux entre une paire respective d'extrémités de conduits et un site commun (S), lesquels conduits servant à transférer un liquide par écoulement capillaire jusqu'au site commun, d'une manière successivement synchronisée à la suite de l'application simultanée du liquide à la paire d'extrémités de conduits, lesdits premier et deuxième conduits se raccordant puis se poursuivant tous deux par des parcours d'écoulement séparés continus.

2. Dispositif selon la revendication 1, dans lequel ledit site commun se trouve dans l'un des parcours d'écoulement continus.

3. Dispositif selon la revendication 1 ou 2, dans lequel lesdits premier et deuxième conduits sont adaptés pour réaliser un régime d'écoulement sensiblement prédéterminé dans la traversée du raccordement.

4. Dispositif selon la revendication 3, dans lequel au moins une partie des premier et deuxième conduits a, dans la direction longitudinale, une résistance hydraulique choisie pour réaliser ledit régime d'écoulement.

5. Dispositif selon la revendication 4, dans lequel au moins une partie des premier et deuxième conduits a une section transversale de dimensions choisies pour réaliser la résistance hydraulique requise.

6. Dispositif selon l'une quelconque des revendications 3 à 5, dans lequel ledit régime d'écoulement est un débit nul dans le raccordement entre lesdits premier et deuxième conduits lorsque les deux conduits sont saturés au moins jusqu'au raccordement.

7. Dispositif selon l'une quelconque des revendications précédentes, dans lequel ledit premier conduit et ledit deuxième conduit comportent respectivement chacun des parties séparées en amont et en aval du raccordement.

8. Dispositif selon la revendication 7, dans lequel, dans chacun des premier et deuxième conduits, le rapport de la résistance hydraulique dans la direction longitudinale dans la partie amont à celle dans la partie aval est sensiblement égal.

9. Dispositif selon la revendication 7, dans lequel au moins une des parties aval desdits premier et deuxième conduits comporte des zones à résistance hydraulique différente dans le sens de l'écoulement.

10. Dispositif selon la revendication 9, dans lequel les zones à résistance hydraulique différente sont agencées pour produire un écoulement oscillant dans la traversée du raccordement entre lesdits premier et deuxième conduits.

11. Dispositif selon l'une quelconque des revendications précédentes, dans lequel au moins un desdits premier et deuxième conduits comporte une pluralité de conduits secondaires disposés en parallèle.

12. Dispositif selon la revendication 11, dans lequel au moins un obstacle transversal à l'écoulement est disposé au voisinage immédiat des extrémités desdits conduits secondaires.

13. Dispositif selon la revendication 12, dans lequel un desdits obstacles transversaux à l'écoulement est disposé pour un sur deux desdits conduits secondaires.

14. Dispositif selon l'une quelconque des revendications précédentes, dans lequel lesdits premier et deuxième conduits comprennent des couches superposées de matière poreuse séparées par une couche imperméable qui est absente là où lesdits conduits se raccordent.

15. Dispositif selon l'une quelconque des revendications 1 à 13, ledit dispositif comportant une seule feuille de matière poreuse et lesdits premier et deuxième conduits étant formés en réalisant dans ladite feuille des zones imperméables de séparation de flux.

16. Dispositif selon la revendication 15, dans lequel ledit premier conduit s'étend depuis ladite extrémité de conduit vers ledit site commun dans une partie disposée globalement au centre de ladite feuille, et ledit deuxième conduit depuis ladite première extrémité avec ledit site commun s'étendant dans deux parties latérales opposées autour de ladite partie centrale, ladite partie centrale et lesdites parties latérales assurant, d'une manière successivement synchronisée, un écoulement de liquide depuis ladite première extrémité jusqu'audit site commun, lesdites parties latérales se raccordant audit premier conduit dans deux zones transversales par rapport auxdites parties latérales et lesdites parties latérales ayant des parties aval au-delà dudit raccordement.

17. Dispositif selon la revendication 16, ledit dispositif étant adapté afin que des flux de liquide provenant des premier et deuxième conduits se rencontrent en un front d'intersection sensiblement rectiligne.

18. Dispositif selon l'une quelconque des revendications précédentes, dans lequel est présent un troisième conduit d'écoulement de liquide raccordé à au moins un desdits premier et deuxième conduits, ledit troisième conduit étant placé de façon qu'un liquide puisse être appliqué simultanément aux extrémités desdits premier, deuxième et troisième conduits.

19. Dispositif selon l'une quelconque des revendications précédentes, dans lequel un réservoir de retardement d'écoulement est disposé dans au moins un desdits premier et deuxième conduits.
